(19) Europäisches Patentamt
European Patent Office
Office européen des brevets

(11) **EP 4 631 530 A1**

(12) **EUROPEAN PATENT APPLICATION**
published in accordance with Art. 153(4) EPC

(43) Date of publication:
**15.10.2025 Bulletin 2025/42**

(21) Application number: **23900637.2**

(22) Date of filing: **05.12.2023**

(51) International Patent Classification (IPC):
**A61L 2/18** (2006.01)        **A01N 43/40** (2006.01)
**A01P 3/00** (2006.01)        **A61L 2/03** (2006.01)
**C02F 1/461** (2023.01)

(52) Cooperative Patent Classification (CPC):
**A01N 43/40; A01P 3/00; A61L 2/03; A61L 2/18;
C02F 1/461**

(86) International application number:
**PCT/JP2023/043383**

(87) International publication number:
**WO 2024/122522 (13.06.2024 Gazette 2024/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.12.2022 JP 2022196933**

(71) Applicant: **National Institute for Materials Science
Tsukuba-shi, Ibaraki 305-0047 (JP)**

(72) Inventors:
• **EMRAN, Mohammed Youssef Mahmoud
Tsukuba-shi, Ibaraki 305-0047 (JP)**
• **OKAMOTO, Akihiro
Tsukuba-shi, Ibaraki 305-0047 (JP)**

(74) Representative: **Haseltine Lake Kempner LLP
One Portwall Square
Portwall Lane
Bristol BS1 6BH (GB)**

(54) **STERILIZATION METHOD AND COMPOSITION USED IN STERILIZATION METHOD**

(57)    A sterilization method for bacteria contained in a biofilm, including: preparing a composition containing an organic compound having the standard oxidation-reduction potential (pH 7) of -0.7 V to -0.2 V, and water; bringing the composition into contact with the biofilm and a conductor; and applying a potential that is higher than the lower limit value of the potential window and that is -0.4 V or lower with respect to a silver/silver chloride electrode to the conductor.

[FIG. 1]

```
  ┌─────────────────────────────┐
  │    PREPARE COMPOSITION      │ ∿ S1
  └─────────────────────────────┘
                │
                ▼
  ┌─────────────────────────────┐
  │ BRING COMPOSITION INTO CONTACT│ ∿ S2
  │   WITH BIOFILM AND CONDUCTOR │
  └─────────────────────────────┘
                │
                ▼
  ┌─────────────────────────────┐
  │      APPLY POTENTIAL        │ ∿ S3
  └─────────────────────────────┘
```

## Description

### TECHNICAL FIELD

The present invention relates to a sterilization method and a composition used in the sterilization method.

### BACKGROUND ART

Currently, due to biofilms created by bacterial populations, management of infectious diseases in healthcare settings has been problematic worldwide.

For bacteria, chemical methods using antibiotics and the like are widely known. However, the appearance of antimicrobial resistant bacteria due to improper use of antibiotics has been problematic. Thus, sterilization techniques other than chemical methods have been studied for many years. For example, in Non-Patent Literature 1 to 4, methods of removing, by generation of $H_2$ and negative-negative surface repulsion through applying a high potential (for example, about 7 V) to a conductor (electrical conductor) such as stainless steel or titanium, a biofilm formed on the surface of the conductor, have been reported.

Citation List

Non-Patent Literature

Non-Patent Literature 1: Current Opinion in Solid State and Materials Science, Vol. 25, Issue 4, August 2021, 100926.

Non-Patent Literature 2: Applied Sciences, 2022, Vol. 12, Issue 13, 6320.

Non-Patent Literature 3: Bioelectrochemistry 121 (2018) p. 84-94.

Non-Patent Literature 4: Colloids and Surfaces B: Niointerfaces, Vol. 117, 1, May 2014. p. 152-157.

### SUMMARY OF INVENTION

Technical Problem

The techniques disclosed in Non-Patent Literatures 1 to 4 do not cause problems of antimicrobial resistant bacteria; however, the techniques need to be performed under severe conditions where electrolysis of a solvent or the like occurs. Sterilization under such severe conditions requires a large amount of energy consumption and is thus costly, and there is also a possibility that active chemical species adversely affecting a human body are generated due to side reactions.

Thus, an object of the present invention is to provide a sterilization method for bacteria contained in a biofilm which can be more efficiently performed under milder conditions and can reduce adverse effects on the human body, and a composition used in the sterilization method.

Solution to Problem

As a result of intensive studies to achieve the above object, the present inventors have found that the above object can be achieved by the following configuration.

[1] A sterilization method for bacteria contained in a biofilm, the method including: preparing a composition containing an organic compound having a standard oxidation-reduction potential (pH 7) of -0.7 V to -0.2 V, and water; bringing the composition into contact with the biofilm and a conductor; and applying a potential that is higher than a lower limit value of a potential window and that is -0.4 V or lower with respect to a silver/silver chloride electrode to the conductor.

[2] The sterilization method according to [1], in which the potential to be applied to the conductor is - 1.2 V to -0.8 V.

[3] The sterilization method according to [1] or [2], in which the organic compound is a compound having a dipyridine structure.

[4] The sterilization method according to [3], in which the organic compound is methyl viologen.

[5] The sterilization method according to any one of [1] to [4], in which the composition further contains a cation.

[6] The sterilization method according to any one of [1] to [5], in which the composition further contains a silver ion.

[7] The sterilization method according to any one of [1] to [6], in which the biofilm is formed on the conductor.

[8] The sterilization method according to any one of [1] to [6], in which the biofilm is formed on a substrate different from the conductor.

[9] The sterilization method according to [8], in which the substrate is an insulator.

[10] The sterilization method according to [9], in which the substrate is polytetrafluoroethylene (PTFE).

[11] The sterilization method according to any one of [7] to [10], in which the conductor on which the biofilm has been formed or the substrate is a medical instrument used in a state where the medical instrument is implanted in a living body or a medical instrument used across or in direct contact with a mucous membrane of a living body.

[12] The sterilization method according to any one of [1] to [11], in which the bacteria are at least one selected from the group consisting of Klebsiella pneumoniae, Staphylococcus epidermidis, and Pseudomonas aeruginosa.

[13] The sterilization method according to any one of [1] to [11], in which the bacteria are Gram-negative rods.

[14] The sterilization method according to [13], in which the Gram-negative rods are bacteria belonging to Enterobacteriaceae.

[15] A composition used in the sterilization method according to any one of [1] to [14] , comprising the organic compound having a standard oxidation-reduction potential of -0.7 V to -0.2 V, and the water.

[16] The composition according to [15], in which the organic compound is a compound having a dipyridine structure.

[17] The composition according to [16], in which the organic compound is methyl viologen.

[18] The composition according to any one of [15] to [17], in which the composition further contains a cation.

[19] The composition according to any one of [15] to [18], in which the composition further contains a silver ion.

Advantageous Effects of Invention

The present invention provides a sterilization method for bacteria contained in a biofilm which can be efficiently performed under mild conditions and can reduce adverse effects on a human body, and a composition used in the sterilization method.

## BRIEF DESCRIPTION OF DRAWINGS

FIG. 1 is a flowchart illustrating a sterilization method of the present embodiment.

FIG. 2 is a schematic diagram illustrating a presumed mechanism of the sterilization method of the present embodiment.

FIG. 3 is a schematic diagram illustrating a presumed mechanism of a sterilization method according to a modification of the present embodiment.

FIG. 4A is a time-current curve (CA diagram) obtained in Experiment 1.

FIG. 4B is a time-current curve (CA diagram) obtained in Experiment 2-1.

FIG. 4C is a diagram showing evaluation results of sterilization effects (the number of colonies) in Experiment 1, Experiment 2-1, and Experiment 3-1.

FIG. 4D is a diagram showing evaluation results of sterilization effects (the amount of bacterial reduction) in Experiment 1 and Experiment 2-1.

FIG. 5A is a diagram showing evaluation results of sterilization effects (the number of colonies) in Experiments 2-1 to 2-4 and Experiments 4-1 to 4-4.

FIG. 5B is a diagram showing evaluation results of sterilization effects (the amount of bacterial reduction) in Experiments 2-1 to 2-4.

FIG. 6 is a diagram showing evaluation results of sterilization effects (the amount of bacterial reduction) in Experiments 5-1 to 5-2.

FIG. 7A is a diagram showing evaluation results of sterilization effects (the amount of bacterial reduction) in Experiments 6-1 to 6-3.

FIG. 7B is a diagram showing evaluation results of sterilization effects (the amount of bacterial reduction) in Experiments 7-1 to 7-2.

FIG. 8A is a diagram showing evaluation results of sterilization effects (the number of colonies) in Experiments 8-1 to 8-9.

FIG. 8B is a diagram showing evaluation results of sterilization effects (the amount of bacterial reduction) in Experiments 8-1 to 8-8.

## DESCRIPTION OF EMBODIMENTS

Hereinafter, the present invention is described in detail.

The description of the components described below may be made based on representative embodiments of the present invention, but the present invention is not limited to such embodiments.

In the present specification, a numerical range represented by "to" means a range including numerical values described before and after "to" as a lower limit value and an upper limit value. That is, "A to B" indicates A or more and B or less.

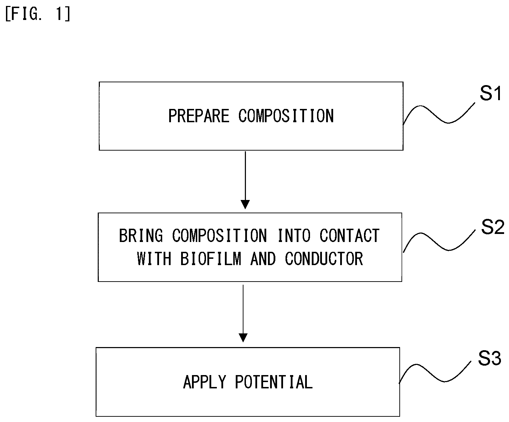

A sterilization method of the present embodiment is described with reference to a flowchart illustrated in FIG. 1. The sterilization method of the present embodiment includes the following steps S1 to S3.

Step S1: a step of preparing a composition containing an organic compound having the standard oxidation-reduction potential (pH 7) of -0.7 V to -0.2 V (hereinafter, appropriately described as "specific organic compound") and water (hereinafter, appropriately described as "sterilizing composition");

step S2: a step of bringing the sterilizing composition into contact with a biofilm and a conductor; and

step S3: a step of applying a potential within a predetermined range to the conductor.

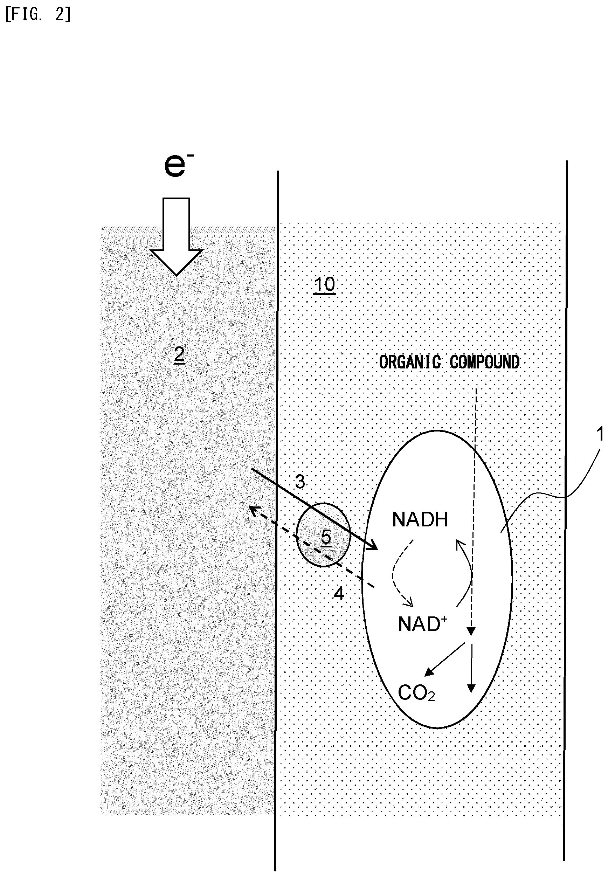

FIG. 2 is a schematic diagram illustrating a presumed mechanism of the sterilization method of the present embodiment. A biofilm 10 containing a bacterial cell (bacterium) 1 is attached onto a conductor 2. In the process in which the bacterial cell 1 decomposes an organic compound to obtain energy (particularly in an anaerobic state), NADH is generated from $NAD^+$ used for decomposition of the organic compound, electrons are transferred from NADH to the conductor 2 as an electron acceptor via an electron transfer system (arrow 4), and NADH returns to $NAD^+$, which is used again to obtain energy.

The inventors have surprisingly found that applying a negative potential to the conductor 2 in a composition containing a specific organic compound 5 can reduce the activity of the bacterial cell 1 in the biofilm 10, and sterilization can be achieved eventually, leading to the present invention. In addition, a weak potential within the range of the potential window is sufficient as the negative potential to be applied to the conductor 2. As a result, sterilization under mild conditions without electrolysis of the solvent (water) is possible. The transfer of electrons (arrow 4) from the bacterial cell 1 to the conductor 2 is limited by applying a negative potential to the conductor 2. In particular, when a potential of -0.4 V or lower with respect to a silver/silver chloride electrode is applied, electrons move from the conductor 2 to the bacterial cell 1 (arrow 3). Then, as electrons move from the conductor 2 to the bacterial cell 1, energy acquisition of the cell is inhibited, the activity further decreases, and finally the bacteria can be killed (sterilized). It is presumed that the specific organic compound 5 as an electron mediator makes electron transfer between the conductor 2 and the bacterial cell 1 efficient. Thus, electron transfer from the conductor 2 to the bacterial cell 1 (arrow 3) becomes smooth, and efficient sterilization can be performed even with application of a weak potential. The present principle of thermodynamically suppressing the metabolism of the bacterium 1 is an innovative one that can be expected to have a continuous effect on antimicrobial resistant bacteria latent in the biofilm 10. Note that the mechanism described above is a presumption, and does not affect the scope of the present invention at all.

Hereinafter, each step of the sterilization method of the present embodiment is described in detail. In the present specification, "sterilization" includes not only eradicating bacteria in a biofilm but also killing some of the bacteria in the biofilm (that is, reducing the number of bacteria in the biofilm).

[Step S1]

Step S1:

First, a sterilizing composition containing an organic compound having the standard oxidation-reduction potential (pH 7) of -0.7 V to -0.2 V (specific organic compound) and water is prepared.

The standard oxidation-reduction potential (pH 7) of the specific organic compound is not particularly limited as long as it is in the range of -0.7 V to -0.2 V, but is preferably -0.6 V to -0.3 V, and more preferably - 0.6 V to -0.4 V from the viewpoint of obtaining a higher sterilization effect.

The specific organic compound is not particularly limited as long as it is an organic compound having the standard oxidation-reduction potential within the above range, but for example, a compound containing a nitrogen-containing aromatic heterocyclic ring, particularly, a compound having a dipyridine structure is preferable. Examples of the specific compounds include compounds shown in Table 1 and derivatives thereof, and among them, from the viewpoint of obtaining a higher sterilization effect, methyl viologen and derivatives thereof are preferable, and methyl viologen is more preferable. The specific organic compound may be composed of a single compound or two or more kinds of compounds.

The standard oxidation-reduction potentials in specific organic compounds can be directly measured by electrochemical measurement by potential sweep using a three-electrode system, and were also determined with reference to the value described in Literature 1 to 3 below.

Literature 1 : Ryo Nakagawa and Yuta Nishina, "Simulating the redox potentials of unexplored phenazine derivatives as electron mediators for biofuel cells" J. Phys. Energy 3 (2021) 034008

Literature 2 : Mary Lou Fultz and Richard A. Durst, "Mediator compounds for the electrochemical study of biological redox systems: a compilation" Analytica Chimica Acta, 140 (1982) 1-18. Elsevier Scientific Publishing Company

Literature 3 : Octavio Reyes-Salas et al. "Titrimetric and Polarographic Determination of Carminic Acid and its Quantification in Cochineal (Dactylopius coccus) Extracts" J. Mex. Chem. Soc. 2011, 55(2), 89-93

[Table 1]

| Specific organic compound | Standard oxidation-reduction potential (pH 7) [V] |
|---|---|
| Anthraquinone-2-sulfonate (AQS) | -0.225 |
| Safranin T | -0.289 |
| Indulin Scarlet (CAS No. 2611-49-6 ) | -0.299 |
| Natural red (CAS No. 553-24-2) | -0.325 |
| 1,1'-Dibenzyl-4,4'-bipyridinium dichloride (benzyl viologen) | -0.358 |

(continued)

| Specific organic compound | Standard oxidation-reduction potential (pH 7) [V] |
|---|---|
| Phenazine-1,6-dicarboxylic acid | -0.36 |
| Indigo carmine | -0.36 |
| 1,1'-Ethylene-2,2'-bipyridylium ion (diquat ion) | -0.361 |
| 1,1'-Bis(2-hydroxyethyl)-4,4'-bipyridinium | -0.408 |
| 1,1'-Dimethyl-4,4'-bipyridinium dichloride (methyl viologen) | -0.440 |
| 1,1'-Diheptyl-4,4' -bipyridinium | -0.48 |
| 1,1'-Diethyle-[4,4'-bipyridine]-1,1'-diium bromide (ethylviologen dibromide) | -0.495 |
| 1,1'-Di isopropyl-4,4'-bipyridinium dichloride | -0.495 |
| 1,1'-Trimethylene-2,2'-bipyridyl | -0.540 |
| 5,5'-Dimethyl-2,2'-bipyridyl | -0.664 |
| Carminic acid (CAMA) | -0.519 |

The content of the specific organic compound in the sterilizing composition is not particularly limited, but is, for example, 1 $\mu$M (M = mol/L) to 1000 $\mu$M, preferably 10 $\mu$M to 200 $\mu$M from the viewpoint of obtaining a higher sterilization effect.

The water contained in the sterilizing composition is not particularly limited, and may be pure water, distilled water, ion-exchanged water, or the like. The content of water in the sterilizing composition is not particularly limited, and is, for example, 90.0 mass% to 99.9 mass% when the total amount of the composition is 100 mass%.

The sterilizing composition may further contain a positive ion (cation). When the specific organic compound and the cation are used in combination, a synergistic effect is produced, and the sterilization effect can be further enhanced. This mechanism is not clear, but is presumed as follows. In the composition containing the specific organic compound 5, transfer of electrons to the bacterium 1 (arrow 3 in FIG. 2) is promoted by applying a negative potential to the conductor 2. At this time, when a cation is contained in the sterilizing composition, the cation flows into the inside of the bacterial cell 1. As a result, an osmotic abnormality occurs in the bacterial cell 1, and the activity of the bacterium 1 decreases, so that sterilization can be achieved eventually. In addition, when the cation has properties of damaging DNA and enzymes in the bacterium 1, the sterilization effect can be further enhanced. It should be noted that the mechanism described above is a presumption, and does not affect the scope of the present invention at all.

The cation is not particularly limited, and as the cation, for example, there may be mentioned a cation containing a metal such as a metal ion or a complex ion, or an organic cation generated from a drug or the like. Examples of the metal contained in the metal ion and the cation containing a metal include silver (Ag), copper (Cu), cobalt (Co), aluminum (Al), nickel (Ni), zinc (Zn), molybdenum (Mo), vanadium (V), zirconium (Zr), tungsten (W), palladium (Pd), platinum (Pt), and the like, and from the viewpoint of further enhancing the sterilization effect, silver ion ($Ag^+$) and copper ions ($Cu^+$, $Cu^{2+}$) are preferable, and silver ($Ag^+$) is more preferable. In addition, the cation may be a so-called cation derived from a physiological electrolyte, and examples of the cation derived from a physiological electrolyte include sodium ion ($Na^+$), potassium ion ($K^+$), calcium ion ($Ca^{2+}$), magnesium ion ($Mg^{2+}$), and the like. As the positive ion (cation), one kind of cation may be used, or two or more kinds of cations may be used.

The sterilizing composition may further contain a negative ion (anion) as a counter ion of the cation. The anion is not particularly limited, and examples thereof include halide ions such as fluorine, chlorine, bromine, iodine, and the like.

The cation may be an ion derived from a salt including a cation and an anion. Preferable examples of the salt include metal halides such as silver chloride and copper chloride. By dispersion or dissolution (including partial dissolution) of these salts in the sterilizing composition, a cation can be introduced into the sterilizing composition.

The concentration of the cation in the sterilizing composition is not particularly limited, and can be appropriately adjusted within the range in which the effect of the present embodiment is exhibited. From the viewpoint of obtaining a higher sterilization effect, for example, the concentration of the cation in the sterilizing composition is preferably 1 $\mu$M to 1 M, and more preferably 10 $\mu$M to 200 mM. The concentration of the anion in the sterilizing composition is not particularly limited, and similarly to the concentration of the cation, from the viewpoint of obtaining a higher sterilization effect, it is preferably 1 $\mu$M to 1 M, and more preferably 10 $\mu$M to 200 mM.

The sterilizing composition may be composed of only the specific organic compound and water, or may contain other components other than the specific organic compound or water within the range in which the effect of the present embodiment is exhibited. Examples of the other components include the above-described cations and anions, and other examples include electron mediators other than specific organic compounds, electron source compounds, buffering agents, coagulants, and the like. The electron source compound is a compound used for metabolism of bacteria, and is not particularly limited, and examples thereof include organic compounds (amino acids, sugars, organic acids, and the like). The buffering agent is not particularly limited, and examples thereof include borate, bicarbonate, Tris-HCl, citrate, phosphate, succinate, phosphate, acetate, and the like. Examples of the coagulant include Kanten (agar), gelatin, agar, and the like and agar is preferable.

The sterilizing composition can be prepared by uniformly mixing a specific organic compound, water, and, if necessary, other components according to a general-purpose method.

In addition, the sterilizing composition may be prepared by adding a predetermined amount of a specific organic compound to a composition containing a general-purpose culture medium (liquid medium, solid medium), a buffer solution, physiological saline, and the like (hereinafter, also referred to as "culture medium composition"). As the media, buffer solutions, physiological saline, and the like, commercially available ones may be used. As the medium, for example, Lysogeny broth (LB medium) is used. The medium composition contains water and the above-described other components. Thus, the sterilizing composition of the present embodiment may be a mixture of a specific organic compound and a culture medium composition. Note that the medium composition need not contain a buffer solution or physiological saline. The content of water contained in the sterilizing composition also includes the water contained in the culture medium composition.

[Step S2]

Step S2:

Next, the sterilizing composition is brought into contact with a biofilm and a conductor.

Bacteria contained in the biofilm, i.e., bacteria to be sterilized in the present embodiment are not particularly limited, and are, for example, any bacteria of Gram-positive bacteria and Gram-negative bacteria. For example, the sterilization method of the present embodiment is also effective against Klebsiella pneumoniae (Gram-negative bacteria), Pseudomonas aeruginosa (Gram-negative bacteria), and Staphylococcus epidermidis (Gram-positive bacteria) defined by the U.S. Food and Drug Administration (FDA) as bacteria that significantly affect endoscopic contamination.

In addition, in the sterilization method of the present embodiment, when the bacteria contained in the biofilm are Gram-negative rods, particularly Gram-negative rods belonging to the Enterobacteriaceae, electron transfer (arrow 3 in FIG. 2) is made more efficient, and a superior sterilization effect is obtained. Examples of bacteria belonging to the Enterobacteriaceae include the above-described Klebsiella pneumoniae (Klebsiella spp.), Enterobacter spp., Escherichia spp., Salmonella spp., Serratia spp., Shigella spp., Yersinia spp., and the like.

The biofilm is a higher order structure formed by bacteria adhered to the surface of a solid phase (substrate), and is covered with, for example, polysaccharides produced by bacteria. As shown in FIG. 2, the biofilm 10 may be formed on a conductor 2 (an example of a substrate).

The material, shape, and size of the conductor are not particularly limited as long as electrons can be exchanged between the conductor and the biofilm. Examples of the material include metal materials such as silver, copper, aluminum, nickel, iron, and alloys containing these metals (for example, stainless steel (SUS)), and carbon materials such as amorphous carbon, graphite, and carbon nanotubes.

Examples of the conductor include a medical instrument used in a state where it is implanted in a living body (medical implant or the like), a medical instrument used beyond a mucous membrane of a living body (forceps or the like), and a medical instrument used in direct contact with a mucous membrane of a living body (endoscope or the like). When this kind of thing is used as the conductor, the sterilization method of the present embodiment can also be utilized as a method for cleaning and washing the medical instrument, the medical implant, or the like.

The method for bringing the sterilizing composition into contact with the biofilm and the conductor is not particularly limited, but for example, when the sterilizing composition is a liquid, a method of immersing the biofilm and the conductor in the sterilizing composition and a method of dropping the sterilizing composition on the biofilm and the conductor can be used. In addition, when the sterilizing composition is solid (for example, in the case of a sterilizing composition in which the medium is solid), there is a method of bringing the sterilizing composition into direct contact with the conductor under pressure or without pressure. Direct contact means that when the conductor is a flat plate, typically the surface on which the biofilm has been formed is brought into contact with the sterilizing composition.

[Step S3]

Step S3:

Next, a potential of higher than the lower limit value of the potential window and -0.4 V or lower with respect to a silver/silver chloride electrode is applied to the conductor.

The inventors have found that, regardless of the kind of bacteria, when the sterilizing composition of the present embodiment is used, electron transfer from the conductor 2 to the bacterial cell 1 (arrow 3 in FIG. 2) is promoted by applying a weak potential of -0.4 V or lower, so that energy acquisition of the cell is inhibited, and sterilization can be achieved eventually. From the viewpoint of obtaining a higher sterilization effect, it is preferable to apply a potential of preferably -0.6 V or lower, more preferably -0.8 V or lower, and still more preferably -0.9 V or lower to the conductor. The lower limit value of the negative potential to be applied to the conductor is not particularly limited as long as it is higher than the lower limit value of the potential window. The lower limit value of the potential window is a value determined by the components of the sterilizing composition, the pH of the sterilizing composition, the material of the electrode, and the like, and is obvious to those skilled in the art. When the potential to be applied is higher than the lower limit value of the potential window, sterilization can be performed under mild conditions in which electrolysis of the solvent (water) does not occur. The sterilization method of the present embodiment is low in cost because energy consumption can be suppressed, and it can also suppress side reactions that may generate active chemical species adversely affecting the human body. Furthermore, in a case in which electrolysis of water is caused by applying a high potential, a reaction such as hydrogen generation is prioritized, and electron transfer from the conductor 2 to the bacterial cell 1 (arrow 3 in FIG. 2) may be suppressed. In the present embodiment, since the potential to be applied is weak, hydrogen or the like is not generated, and sterilization can be achieved by efficiently suppressing the metabolism of the bacterium 1. The lower limit value of the potential to be applied to the conductor is, for example, -1.4 V or more, preferably -1.2 V or more, and more preferably -1.1 V or more with respect to a silver/silver chloride electrode.

A method of applying a potential to the conductor is not particularly limited. For example, there is a method in which a working electrode, a reference electrode, and a counter electrode are brought into contact with the sterilizing composition, and in a state where the working electrode is into contact with the above-described conductor, a set of these electrodes (working electrode, counter electrode, and reference electrode) is connected to a potentiostat, which is controlled to apply a predetermined potential to the working electrode. The materials of the working electrode, the counter electrode, and the reference electrode are not particularly limited, and a working electrode, a counter electrode, and a reference electrode known for electrochemical measurement or the like can be used. The reference electrode is preferably a silver/silver chloride electrode. In addition, instead of separately preparing a working electrode, a conductor on which a biofilm has been formed may be used as the working electrode.

The sterilizing composition may be a liquid or a solid, but when it is a liquid, the electrode set (working electrode, counter electrode, and reference electrode) may be immersed in the sterilizing composition. Alternatively, the sterilizing composition may be added dropwise so as to touch the electrode set produced by printed electronics or the like. When the sterilizing composition is solid, the working electrode, the counter electrode, and the reference electrode may be brought into direct contact with the sterilizing composition, similarly to the above conductor.

This step (step S3) may be performed under anaerobic conditions not containing oxygen (for example, a nitrogen atmosphere) or under aerobic conditions containing oxygen (for example, in the atmosphere). Since the atmosphere is not limited to anaerobic conditions, the sterilization method of the present embodiment can be carried out with simpler equipment.

The temperature during this step (step S3) (sterilization temperature) is not particularly limited, and may be, for example, 0°C to 100°C or room temperature. In addition, the potential application time (sterilization time) is not particularly limited, and may be appropriately adjusted according to the kind of the specific organic compound, the kind of bacteria, the kind and size of the conductor, and the like, but may be, for example, 30 minutes to 24 hours.

According to the sterilization method of the present embodiment described above, it is possible to reduce the activity of bacteria in the biofilm and finally kill (sterilize) all or some of the bacteria in the biofilm only by applying a weak potential to the conductor. Thus, a continuous effect on antimicrobial resistant bacteria latent in biofilms can be expected. In addition, by using the sterilization method of the present embodiment, an antibacterial agent is unnecessary, or the amount of the antibacterial agent to be used can be reduced, so that generation of new antimicrobial resistant bacteria can also be suppressed.

Furthermore, when the conductor is a medical instrument, a medical implant, or the like, the sterilization method of the present embodiment can kill bacteria in the biofilm even if mechanical cleaning is difficult due to a complicated shape or the like, and thus the effect of preventing bacterial infection can be expected. Moreover, when the conductor is a medical instrument used in a state where it is implanted in a living body (for example, various implants such as dental implants) or a medical instrument used beyond or in direct contact with a mucous membrane of a living body (for example, forceps, an endoscope, and the like), a biofilm may be generated in fine parts where mechanical washing cannot reach. Bacteria in such biofilms can also be killed (sterilized) by the sterilization method of the present embodiment.

<Modification>

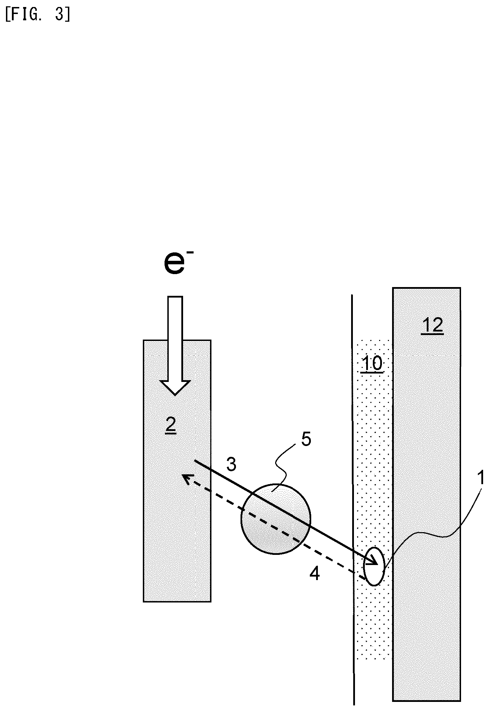

In the above-described embodiment, as shown in FIG. 2, the biofilm 10 is formed on the conductor 2, but the present invention is not limited thereto. For example, as illustrated in FIG. 3, the biofilm 10 may be formed on a substrate 12 different from the conductor 2. The sterilization method described in this modification is the same as the sterilization method described in the above embodiment except that the biofilm 10 is formed on the substrate 12, and the same effect is obtained. Description of similar contents is omitted.

Also in the present modification, the specific organic compound 5 makes electron transfer from the conductor 2 to the bacterial cell 1 (arrow 3 in FIG. 3) efficient. Thus, by applying a negative potential to the conductor 2, the bacterium 1 in the biofilm 10 on the substrate 12 can be killed (sterilized) without directly applying a potential to the substrate 12.

The material of the substrate 12 is not particularly limited, but since it is not necessary to apply a potential, an insulator such as a resin (plastic) can be used. Examples of the resin include polytetrafluoroethylene (PTFE), polyethylene terephthalate (PET), polycarbonate (PC), polyvinyl chloride (PVC), polypropylene (PP), polyethylene (PE), polystyrene (PS), and the like.

Examples of the substrate 12 include medical instruments used in a state where it is implanted in a living body (medical implants and the like), medical instruments used beyond a mucous membrane of a living body (forceps and the like), and medical instruments used in direct contact with a mucous membrane of a living body (endoscopes and the like). When such instruments areused as the substrate 12, the sterilization method of the present modification can also be utilized as a method for cleaning and washing the medical instrument, the medical implant, or the like.

In the present modification, the conductor 2 may be, for example, an electrode (working electrode) itself.

The conductor 2 and the substrate 12 may be in a non-contact state as illustrated in FIG. 3 or may be in contact with each other. The shortest distance between the conductor 2 and the substrate 12 may be, for example, 0 (contact state) to 1 m. When the shortest distance between the conductor 2 and the substrate 12 is within the above range, the bacterium 1 in the biofilm 10 can be efficiently killed (sterilized).

According to the sterilization method of the present modification, it is possible to sterilize a biofilm formed on an insulator (substrate 12) such as PTFE to which a potential cannot be applied. Thus, the sterilization method of the present modification can be applied to sterilization and washing of a medical instrument made of an insulator.

Examples

Hereinafter, the present invention is described in more detail based on examples. Materials, amounts used, proportions, processing contents, processing procedures, and the like shown in the following examples can be appropriately changed without departing from the gist of the present invention. Thus, the scope of the present invention should not be interpreted in any way limited by the following examples.

[Experiment 1]

In this experiment, a stainless steel (SUS304) tube was used as a conductor to sterilize Klebsiella pneumoniae (KP) in a biofilm formed on the SUS tube. As the specific organic compound, methyl viologen (MV) was used.

(1) Biofilm formation

Klebsiella pneumoniae (KP) was added to TSB medium (tryptone soy broth medium), and the cell optical density was adjusted to $OD_{600 nm} = 1.0$. The SUS tube was immersed in this medium, and incubated at 37°C for 24 hours. In this way, a biofilm containing Klebsiella pneumoniae (KP) on the SUS tube surface was formed.

(2) Preparation of sterilizing composition (preparation)

Methyl viologen (MV) was added to DM (defined medium, liquid medium) such that the concentration becomes 100 μM, and the mixture was uniformly mixed. The standard oxidation-reduction potential (pH 7) of methyl viologen is -0.440 V as shown in Table 1.

(3) Potential application

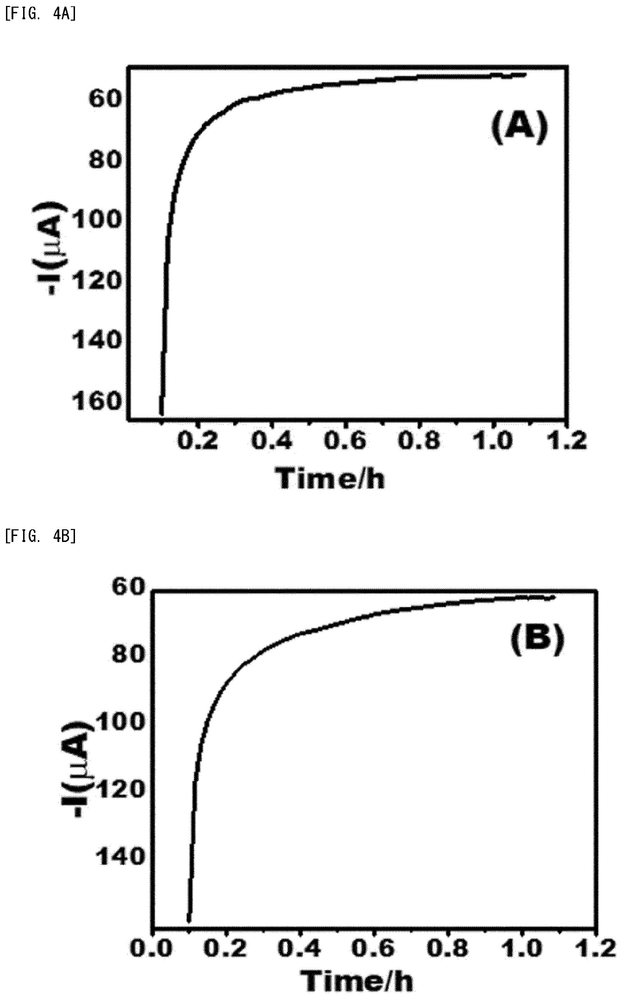

A three-electrode electrochemical cell having a titanium (Ti) wire as a working electrode, a platinum (Pt) wire as a counter electrode, and a silver/silver chloride electrode as a reference electrode was prepared, and an SUS tube on which the biofilm had been formed was electrically connected to the working electrode (titanium wire). The MV-containing medium (DM) was poured into the three-electrode electrochemical cell, the MV-containing medium (DM) was brought into contact with the electrode set (working electrode, counter electrode, and reference electrode) and the SUS tube on which the biofilm had been formed, the potential of the working electrode was set to -1.0 V (vs Ag/AgCl) at room temperature, and the chronoamperometry (CA) was measured for 1 hour under anaerobic conditions (nitrogen atmosphere). That is, a potential was applied to the SUS tube via the working electrode. FIG. 4A shows a time-current curve (CA diagram).

[Experiment 2-1]

In this experiment, an experiment was performed in the same manner as in Experiment 1 except that a potential was applied under aerobic conditions (in the atmosphere). FIG. 4B shows a time-current curve (CA diagram).

[Experiment 2-2] to [Experiment 2-4]

In Experiments 2-2 to 2-4, experiments were performed in the same manner as in Experiment 2-1 except that another strain of Klebsiella pneumoniae (KPt), Staphylococcus epidermidis (SE), and Pseudomonas aeruginosa (PA), respectively, were used instead of Klebsiella pneumoniae (KP). That is, in Experiments 2-2 to 2-4, a potential was applied under aerobic conditions (in the air) as in Experiment 2-1.

[Experiment 3-1]

In this experiment, no methyl viologen (MV) was used, and no potential was applied. First, a biofilm containing KP was formed on a SUS tube in the same manner as in Experiment 1. Then, the SUS tube on which the biofilm had been formed was washed three times with a phosphate buffer solution (PBS), and then incubated in DM (not containing MV) at room temperature for 1 hour without applying a potential.

[Experiment 3-2] to [Experiment 3-4]

In Experiments 3-2 to 3-4, experiments were performed in the same manner as in Experiment 3-1 except that another strain of Klebsiella pneumoniae (KPt), Staphylococcus epidermidis (SE), and Pseudomonas aeruginosa (PA), respectively, were used instead of Klebsiella pneumoniae (KP). That is, in Experiments 3-2 to 3-4, methyl viologen (MV) was not used, and no potential was applied.

[Experiment 4-1]

In this experiment, methyl viologen (MV) was used, but no potential was applied.
First, a biofilm containing KP was formed on a SUS tube in the same manner as in Experiment 1. Then, the SUS tube on which the biofilm had been formed was washed three times with PBS, and then incubated in DM containing methyl viologen (MV) at a concentration of 100 $\mu$M at room temperature for 1 hour without applying a potential.

[Experiment 4-2] to [Experiment 4-4]

In Experiments 4-2 to 4-4, experiments were performed in the same manner as in Experiment 4-1 except that another strain of Klebsiella pneumoniae (KPt), Staphylococcus epidermidis (SE), and Pseudomonas aeruginosa (PA), respectively, were used instead of Klebsiella pneumoniae (KP). That is, in Experiments 4-2 to 4-4, the specific organic compound (MV) was used, but no potential was applied.

[Method for evaluating sterilization effect]

In each experiment, the SUS tube was taken out from DM (liquid medium) and washed three times with PBS (pH = 7.2). The washed SUS tube and 1 mL of PBS were placed in a container, and subjected to strong shaking (30 seconds) by a vortex shaker, ultrasonic treatment (7 minutes), and strong shaking again (30 seconds) to peel the biofilm from the SUS tube. PBS containing the peeled biofilm was diluted 100-fold. Two samples in which 100 $\mu$L of diluted biofilm-containing PBS was spread on a TSB-Kanten (agar) medium plate were prepared and incubated overnight at 37°C. After incubation, the number of colonies formed in each of the two samples was counted and the average was determined. From the average value, the number of colonies (colony forming unit (CFU)/mL) in each experiment was determined.

[Evaluation results]

<Influence of atmosphere on sterilization effect>

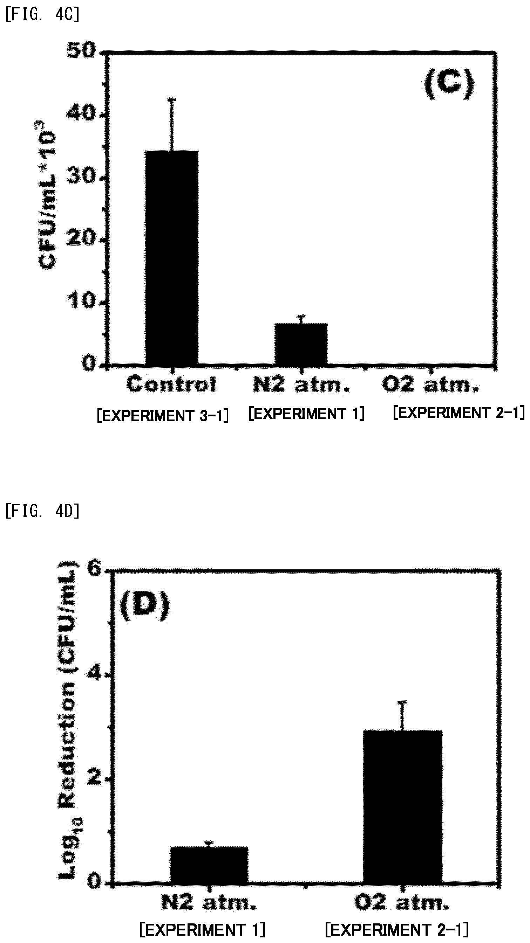

FIG. 4C shows the results (number of colonies) of Experiment 1 (MV, potential application under anaerobic conditions), Experiment 2-1 (MV, potential application under aerobic conditions), and Experiment 3-1 (control, no MV, no potential application).

The number of bacterial colonies was reduced in Experiments 1 and 2-1 as compared with Experiment 3-1 (control). From this, it was found that the bacteria in the biofilm can be sterilized by applying a potential to the SUS tube in the composition containing MV regardless of the atmosphere at the time of applying the potential. In particular, in Experiment 2-1 where the potential was applied under aerobic conditions, it was possible to reduce the number of bacteria dramatically.

In order to more clearly compare the results of Experiments 1 and 2-1, FIG. 4D shows the amount of bacterial reduction in each of Experiments 1 and 2-1. The amount of bacterial reduction is the difference obtained by subtracting the number of colonies in each experiment from the number of colonies in Experiment 3-1 (control) shown in FIG. 4C. As shown in FIG. 4D, a 100-fold or more sterilization effect was obtained under the aerobic conditions (Experiment 2-1) as compared with under the anaerobic conditions (Experiment 1).

<Sterilization effect in biofilm formed by various bacteria>

Table 2 shows the sterilization efficiency in Experiments 2-1 to 2-4 (MV, potential application under aerobic conditions) each using a different kind of bacteria. When the number of colonies in each Experiments 2-1 to 2-4 is denoted by "X" and the number of colonies in each Experiments 3-1 to 3-4 (control, no MV, no potential application) using the same inoculum corresponding thereto is denoted by "Y", the sterilization efficiency is expressed by the following formula.

$$\text{Sterilization efficiency (\%)} = \{(Y - X)/Y)\} \times 100$$

[Table 2]

| Experiment (potential application under aerobic conditions) | Bacteria | Sterilization efficiency (%) |
|---|---|---|
| 2-1 | KP | 100 |
| 2-2 | KPt | 98.7 |
| 2-3 | SE | 98.6 |
| 2-4 | PA | 100 |

As shown in Table 2, regardless of the kind of bacteria, high sterilization efficiency of 95% or more was obtained in a sterilization time as short as 1 hour in any of Experiments 2-1 to 2-4.

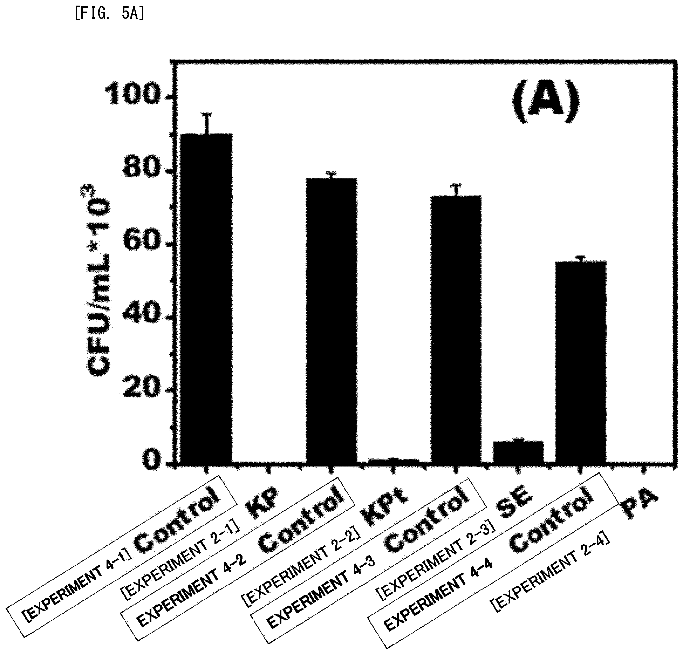

In addition, FIG. 5A shows the results of Experiments 2-1 to 2-4 and Experiments 4-1 to 4-4 (control, MV, no potential application) using each inoculum corresponding thereto. Regardless of the kind of bacteria, the number of bacterial colonies was significantly reduced in any of Experiments 2-1 to 2-4 as compared with Experiments 4-1 to 4-4 (control). From this result, it was possible to confirm that the sterilization effect was not obtained by simply immersing the biofilm in the composition containing MV, but the sterilization effect was obtained by applying a potential to the SUS tube.

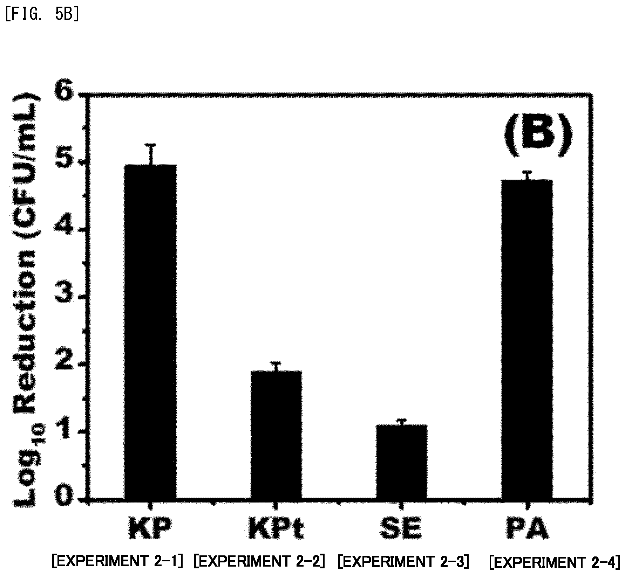

In order to more clearly compare the results of Experiments 2-1 to 2-4, FIG. 5B shows the amount of bacterial reduction in each of Experiments 2-1 to 2-4. The amount of bacterial reduction is the difference obtained by subtracting the number of colonies of the corresponding Experiments 2-1 to 2-4 from the number of colonies in Experiments 4-1 to 4-4 (control) shown in FIG. 5A. As shown in FIG. 5B, the sterilization effects on Gram-negative bacteria (KP, KPt, PA) higher than that on Gram-positive bacteria (SE) were obtained.

[Experiment 5-1]

In this experiment, an experiment was performed in the same manner as in Experiment 2-1 except that a PTFE tube, which is an insulator, was used instead of the SUS tube. That is, in this experiment, a biofilm containing Klebsiella pneumoniae (KP) was formed on a PTFE tube, and a potential was applied under aerobic conditions (in the air) to the working electrode WE (titanium wire) in contact with the PTFE tube in DM (liquid medium) containing methyl viologen (MV). In this experiment, the working electrode (titanium wire) itself corresponds to a "conductor" to which a potential is applied.

[Experiment 5-2]

In this experiment, an experiment was performed in the same manner as in Experiment 5-1 except that no potential was applied. That is, in this experiment, a PTFE tube with biofilm containing Klebsiella pneumoniae (KP) was incubated at room temperature for 1 hour in DM containing methyl viologen (MV) without applying a potential.

[Experiment 5-3]

In this experiment, first, a biofilm containing KP was formed on a PTFE tube in the same manner as in Experiment 5-1. In this experiment, no further operation was performed.

[Evaluation of sterilization effect]

For Experiments 5-1 and 5-2, the number of colonies (colony forming units (CFU/mL)) in each experiment was determined in the same manner as in the "Method for evaluating sterilization effect" described above.
Also in Experiment 5-3, the PTFE tube on which the biofilm had been formed was taken out from the TSB medium, and then the number of colonies (colony forming units (CFU/mL)) in each experiment was determined in the same manner as in the "Method for evaluating sterilization effect" described above.

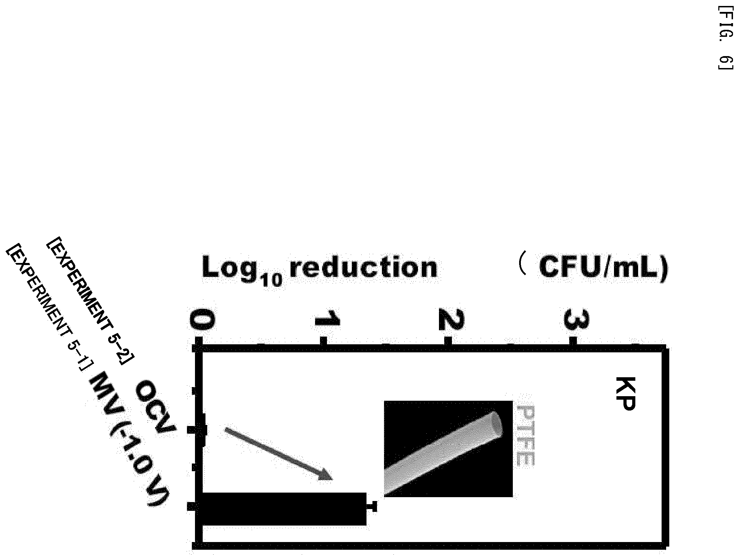

FIG. 6 shows the amount of bacterial reduction in each of Experiments 5-1 and 5-2. The amount of bacterial reduction is the difference obtained by subtracting the number of colonies in each experiment from the number of colonies in Experiment 5-3 (control). As shown in FIG. 6, the amount of bacterial reduction in Experiment 5-1 (MV, potential application) was much larger than that in Experiment 5-2 (MV, no potential application). From this result, it was possible to confirm that even when the substrate on which the biofilm had been formed was an insulator (PTFE), a high sterilization effect was obtained by applying a potential to the conductor in the sterilizing composition.

[Experiment 6-1]

In this experiment, an experiment was performed in the same manner as in Experiment 5-1 except that Pseudomonas aeruginosa (PA) was used instead of Klebsiella pneumoniae (KP). That is, in this experiment, Pseudomonas aeruginosa (PA) in the biofilm formed on the PTFE tube was sterilized in DM (liquid medium) containing methyl viologen (MV).

[Experiment 6-2]

In this experiment, DM (liquid medium) containing 100 $\mu$M of methyl viologen (MV) and 20 $\mu$M of silver chloride was used as a sterilizing composition. The experiment was performed in the same manner as in Experiment 6-1 except for the above.

[Experiment 6-3]

In this experiment, DM (liquid medium) containing 20 $\mu$M of silver chloride (AgCl) instead of methyl viologen (MV) was used as a sterilizing composition. The experiment was performed in the same manner as in Experiment 6-1 except for the above.

[Experiment 6-4]

In this experiment, methyl viologen (MV) and silver chloride were not used, and no potential was applied. The experiment was performed in the same manner as in Experiment 6-1 except for the above. That is, in this experiment, a PTFE tube with biofilm containing Pseudomonas aeruginosa (PA) was incubated in DM at room temperature for 1 hour without applying a potential.

[Experiment 7-1]

In this experiment, an experiment was performed in the same manner as in Experiment 5-1 except that Staphylococcus epidermidis (SE) was used instead of Klebsiella pneumoniae (KP). That is, in this experiment, the sterilization of Staphylococcus epidermidis (SE) in the biofilm formed on the PTFE tube was performed in DM (liquid medium) containing methyl viologen (MV).

[Experiment 7-2]

In this experiment, DM (liquid medium) containing 100 $\mu$M of methyl viologen (MV) and 20 $\mu$M of silver chloride was used as a sterilizing composition. The experiment was performed in the same manner as in Experiment 7-1 except for the above.

[Experiment 7-3]

In this experiment, methyl viologen (MV) and silver chloride were not used, and no potential was applied. The experiment was performed in the same manner as in Experiment 7-1 except for the above. That is, in this experiment, a PTFE tube with biofilm containing Staphylococcus epidermidis (SE) was incubated at room temperature for 1 hour in DM without applying a potential.

[Evaluation of sterilization effect]

For Experiments 6-1 to 6-4 and 7-1 to 7-3, the number of colonies (colony forming units (CFU/mL)) in each experiment was determined in the same manner as in the "Method for evaluating sterilization effect" described above.

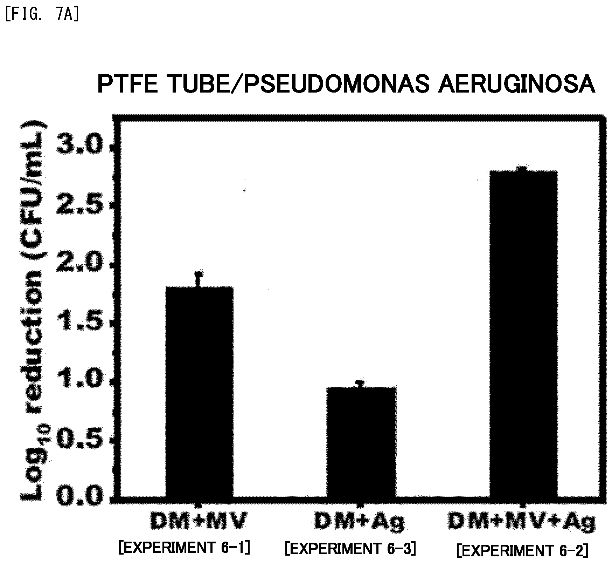

FIG. 7A shows the amount of bacterial reduction in each of Experiments 6-1 to 6-3 using Pseudomonas aeruginosa. The amount of bacterial reduction is the difference obtained by subtracting the number of colonies in each experiment from the number of colonies in Experiment 6-4 (control). As seen from FIG. 7A, the amounts of bacterial reduction in both Experiment 6-1 (MV only) and Experiment 6-2 (combination of MV and AgCl) were larger than that in Experiment 6-3 (AgCl only). Furthermore, the amount of bacterial reduction in Experiment 6-2 (combination of MV and AgCl) was even larger than that in Experiment 6-1 (MV only). From the above results, it was possible to confirm that the sterilizing composition can obtain a high sterilization effect also on Pseudomonas aeruginosa (PA), and further, it was possible to confirm that when MV and Ag$^+$ are used in combination in the sterilizing composition, a synergistic effect is produced, and a higher sterilization effect can be obtained.

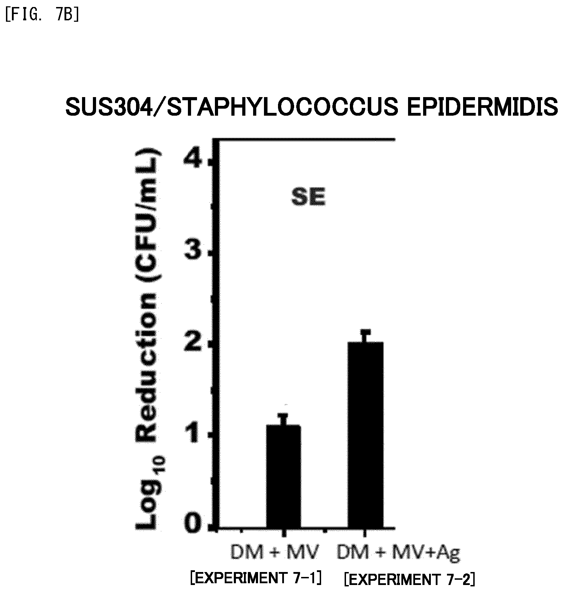

FIG. 7B shows the amount of bacterial reduction in each of Experiments 7-1 and 7-2 using Staphylococcus epidermidis (SE). The amount of bacterial reduction is the difference obtained by subtracting the number of colonies in each experiment from the number of colonies in Experiment 7-3 (control). As seen from the results of Experiment 7-1 shown in FIG. 7B, it was possible to confirm the sterilization effect on Staphylococcus epidermidis (SE) (Experiment 7-1) on the insulating substrate (PTFE) similarly to that on Klebsiella pneumoniae (KP) (FIG. 6, Experiment 5-1) and Pseudomonas aeruginosa (PA) (FIG. 7A, Experiment 6-1) on the insulating substrate (PTFE). In addition, the amount of bacterial reduction in Experiment 7-2 (combination of MV and AgCl) was larger than that in Experiment 7-1 (MV only). Also from this result, it was possible to confirm that a higher sterilization effect can be obtained by using MV and Ag$^+$ in combination in the sterilizing composition.

[Experiment 8-1]

In this experiment, an experiment was performed in the same manner as in Experiment 2-1 except that carminic acid (CAMA) was used instead of methyl viologen (MV). That is, Klebsiella pneumoniae (KP) in biofilms formed on the SUS tube surface was sterilized under aerobic conditions (in air). However, the biofilm was formed by adding Klebsiella pneumoniae (KP) to TSB medium, adjusting the cell optical density to be $OD_{600\,nm} = 0.01$, immersing the SUS tube in this medium, and incubating it at 37°C for 3 days. The standard oxidation-reduction potential (pH 7) of carminic acid is -0.519 V as shown in Table 1.

[Experiment 8-2]

In this experiment, an experiment was performed in the same manner as in Experiment 8-1 except that anthraquinone-2-sulfonate (AQS) was used instead of carminic acid (CAMA). The standard oxidation-reduction potential (pH 7) of anthraquinone-2-sulfonate is -0.225 V as shown in Table 1.

[Experiment 8-3]

In this experiment, an experiment was performed in the same manner as in Experiment 8-1 except that 1,1'-diheptyl-4,4'-bipyridinium (DBPDB) was used instead of carminic acid (CAMA). The standard oxidation-reduction potential (pH 7) of 1,1'-diheptyl-4,4'-bipyridinium is -0.48 V as shown in Table 1.

[Experiment 8-4]

In this experiment, an experiment was performed in the same manner as in Experiment 8-1 except that benzyl viologen (BV) was used instead of carminic acid (CAMA). The standard oxidation-reduction potential (pH 7) of benzyl viologen is -0.358 V as shown in Table 1.

[Experiment 8-5] to [Experiment 8-8]

Experiment 8-5 to Experiment 8-8 are experiments respectively corresponding to Experiment 8-1 to Experiment 8-4, and specific organic compounds which were the same as those in Experiment 8-1 to Experiment 8-4 were used, but no potential was applied.

[Experiment 8-9]

In this experiment (control), no specific organic compound was used, and no potential was applied. A biofilm containing KP was formed on a SUS tube in the same manner as in Experiment 8-1. Then, the SUS tube on which the biofilm had been formed was incubated in DM (containing no specific organic compound) at room temperature for 1 hour without applying a potential.

[Method for evaluating sterilization effect]

In each experiment, the SUS tube was taken out from DM (liquid medium) and washed three times with PBS (pH = 7.2). The washed SUS tube and 1 mL of PBS were placed in a container, and subjected to strong shaking (30 seconds) by a vortex shaker, ultrasonic treatment (7 minutes), and strong shaking again (30 seconds) to peel the biofilm from the SUS tube. PBS containing the peeled biofilm was diluted 100-fold. Three samples in which 100 $\mu$L of diluted biofilm-containing PBS was spread on a TSB-Kanten (agar) medium plate were prepared and incubated overnight at 37°C. After incubation, the number of colonies formed in each of the three samples was counted, and the average value was determined. From the average value, the number of colonies (colony forming unit (CFU)/mL) in each experiment was determined.

[Evaluation results]

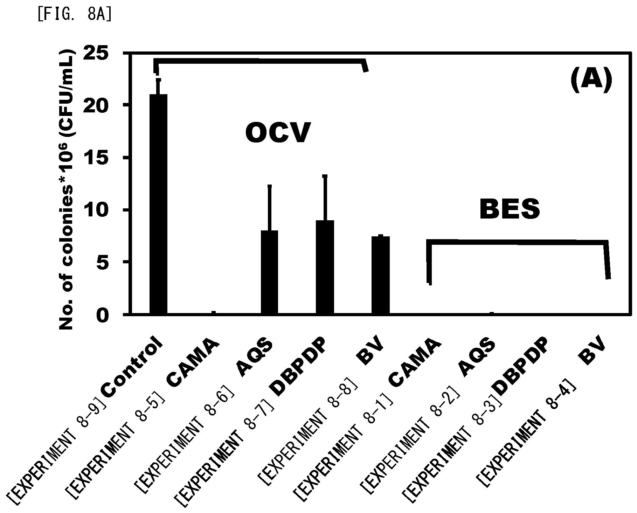

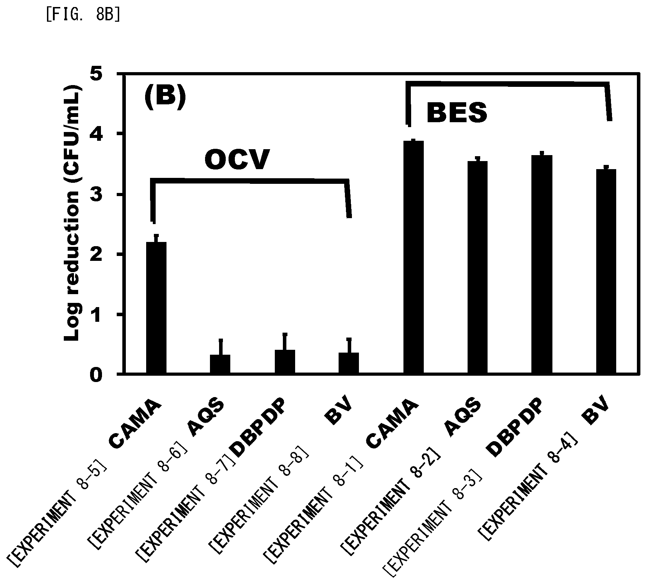

FIG. 8A shows the number of colonies for Experiments 8-1 to 8-9. FIG. 8B shows the amount of bacterial reduction in each of Experiments 8-1 to 8-8. The amount of bacterial reduction is the difference obtained by subtracting the number of colonies in each experiment from the number of colonies in Experiment 8-9 (control) shown in FIG. 8A. As shown in FIG. 8A and FIG. 8B, it was possible to confirm that in Experiments 8-1 to 8-4 (specific organic compound, potential application), the amounts of bacterial reduction were larger than those in Experiments 8-5 to 8-8 (specific organic compound, no potential application), and higher sterilization effects were obtained.

Industrial Applicability

The sterilization method of the present invention can be used for washing and cleaning for preventing infectious diseases caused by medical instruments and medical implants.

Reference Signs List

1    Bacterial cell (bacterium)
2    Conductor
5    Specific organic compound
10   Biofilm
12   Substrate

**Claims**

**1.** A sterilization method for bacteria contained in a biofilm, the method comprising:

preparing a composition containing an organic compound having a standard oxidation-reduction potential (pH 7) of -0.7 V to -0.2 V, and water;

bringing the composition into contact with the biofilm and a conductor; and

applying a potential that is higher than a lower limit value of a potential window and that is -0.4 V or lower with respect to a silver/silver chloride electrode to the conductor.

2. The sterilization method according to claim 1, wherein the potential to be applied to the conductor is - 1.2 V to -0.8 V.

3. The sterilization method according to claim 1 or 2, wherein the organic compound is a compound having a dipyridine structure.

4. The sterilization method according to claim 3, wherein the organic compound is methyl viologen.

5. The sterilization method according to any one of claims 1 to 4, wherein the composition further contains a cation.

6. The sterilization method according to any one of claims 1 to 5, wherein the composition further contains a silver ion.

7. The sterilization method according to any one of claims 1 to 6, wherein the biofilm is formed on the conductor.

8. The sterilization method according to any one of claims 1 to 6, wherein the biofilm is formed on a substrate different from the conductor.

9. The sterilization method according to claim 8, wherein the substrate is an insulator.

10. The sterilization method according to claim 9, wherein the substrate is polytetrafluoroethylene (PTFE).

11. The sterilization method according to any one of claims 7 to 10, wherein the conductor on which the biofilm has been formed or the substrate is a medical instrument used in a state where the medical instrument is implanted in a living body or a medical instrument used across or in direct contact with a mucous membrane of a living body.

12. The sterilization method according to any one of claims 1 to 11, wherein the bacteria are at least one selected from the group consisting of Klebsiella pneumoniae, Staphylococcus epidermidis, and Pseudomonas aeruginosa.

13. The sterilization method according to any one of claims 1 to 11, wherein the bacteria are Gram-negative rods.

14. The sterilization method according to claim 13, wherein the Gram-negative rods are bacteria belonging to Enterobacteriaceae.

15. A composition used in the sterilization method according to any one of claims 1 to 14, comprising the organic compound having a standard oxidation-reduction potential of -0.7 V to -0.2 V and the water.

16. The composition according to claim 15, wherein the organic compound is a compound having a dipyridine structure.

17. The composition according to claim 16, wherein the organic compound is methyl viologen.

18. The composition according to any one of claims 15 to 17, wherein the composition further contains a cation.

19. The composition according to any one of claims 15 to 18, wherein the composition further contains a silver ion.

[FIG. 1]

```
┌─────────────────────────────────────┐
│                                      │        S1
│        PREPARE COMPOSITION           │╮╱
│                                      │╯
└─────────────────────────────────────┘
                    │
                    ▼
┌─────────────────────────────────────┐
│  BRING COMPOSITION INTO CONTACT      │╮╱     S2
│    WITH BIOFILM AND CONDUCTOR        │╯
│                                      │
└─────────────────────────────────────┘
                    │
                    ▼
┌─────────────────────────────────────┐
│                                      │        S3
│         APPLY POTENTIAL              │╮╱
│                                      │╯
└─────────────────────────────────────┘
```

[FIG. 2]

[FIG. 3]

[FIG. 4A]

[FIG. 4B]

[FIG. 4C]

[EXPERIMENT 3-1]    [EXPERIMENT 1]    [EXPERIMENT 2-1]

[FIG. 4D]

[EXPERIMENT 1]    [EXPERIMENT 2-1]

[FIG. 5A]

[FIG. 5B]

[EXPERIMENT 2-1] [EXPERIMENT 2-2] [EXPERIMENT 2-3] [EXPERIMENT 2-4]

[FIG. 6]

[FIG. 7A]

PTFE TUBE/PSEUDOMONAS AERUGINOSA

DM+MV
[EXPERIMENT 6-1]

DM+Ag
[EXPERIMENT 6-3]

DM+MV+Ag
[EXPERIMENT 6-2]

[FIG. 7B]

## SUS304/STAPHYLOCOCCUS EPIDERMIDIS

[FIG. 8A]

[FIG. 8B]

# EP 4 631 530 A1

## INTERNATIONAL SEARCH REPORT

International application No.

**PCT/JP2023/043383**

### A. CLASSIFICATION OF SUBJECT MATTER

*A61L 2/18*(2006.01)i; *A01N 43/40*(2006.01)i; *A01P 3/00*(2006.01)i; *A61L 2/03*(2006.01)i; *C02F 1/461*(2023.01)i
FI:  A61L2/18; A01P3/00; A61L2/03; C02F1/461 Z; A01N43/40 102

According to International Patent Classification (IPC) or to both national classification and IPC

### B. FIELDS SEARCHED

Minimum documentation searched (classification system followed by classification symbols)

A61L2/00; A01N43/40; A01P3/00; C02F1/461

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | US 2015/0073491 A1 (THE RESEARCH FOUNDATION FOR THE STATE UNIVERSITY OF NEW YORK) 12 March 2015 (2015-03-12) paragraphs [0028], [0037], [0041]-[0043], [0052], [0053], [0060], [0063], fig. 8 | 1-5, 7-18 |
| A | same as above | 6, 19 |
| Y | JP 6-285138 A (MATSUNAGA, Tadashi) 11 October 1994 (1994-10-11) claim 1, paragraphs [0002], [0003], [0010] | 1-5, 7-18 |
| A | JP 2012-34576 A (KAJIMA CORP.) 23 February 2012 (2012-02-23) paragraph [0038] | 4, 17 |
| A | US 5312813 A (UNIVERSITY TECHNOLOGIES INTERNATIONAL) 17 May 1994 (1994-05-17) claims | 1-19 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **11 January 2024** | **23 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

INTERNATIONAL SEARCH REPORT
Information on patent family members

International application No.

**PCT/JP2023/043383**

| Patent document cited in search report | | | Publication date (day/month/year) | Patent family member(s) | | | Publication date (day/month/year) |
|---|---|---|---|---|---|---|---|
| US | 2015/0073491 | A1 | 12 March 2015 | US | 2011/0029080 | A1 | |
| | | | | US | 2017/0000918 | A1 | |
| | | | | US | 2019/0105414 | A1 | |
| | | | | US | 2015/0076000 | A1 | |
| | | | | WO | 2013/159107 | A1 | |
| JP | 6-285138 | A | 11 October 1994 | (Family: none) | | | |
| JP | 2012-34576 | A | 23 February 2012 | (Family: none) | | | |
| US | 5312813 | A | 17 May 1994 | WO | 1992/019286 | A1 | |
| | | | | AU | 1672492 | A | |
| | | | | CA | 2109084 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Current Opinion in Solid State and Materials Science*, August 2021, vol. 25 (4), 100926
- *Applied Sciences*, 2022, vol. 12 (13), 6320
- *Bioelectrochemistry*, 2018, vol. 121, 84-94
- *Colloids and Surfaces B: Niointerfaces*, 01 May 2014, vol. 117, 152-157
- **RYO NAKAGAWA** ; **YUTA NISHINA**. Simulating the redox potentials of unexplored phenazine derivatives as electron mediators for biofuel cells. *J. Phys. Energy*, 2021, vol. 3, 034008
- Mediator compounds for the electrochemical study of biological redox systems: a compilation. **MARY LOU FULTZ** ; **RICHARD A. DURST**. Analytica Chimica Acta. Elsevier Scientific Publishing Company, 1982, vol. 140, 1-18
- **OCTAVIO REYES-SALAS et al.** Titrimetric and Polarographic Determination of Carminic Acid and its Quantification in Cochineal (Dactylopius coccus) Extracts. *J. Mex. Chem. Soc.*, 2011, vol. 55 (2), 89-93
- *CHEMICAL ABSTRACTS*, 2611-49-6
- *CHEMICAL ABSTRACTS*, 553-24-2